# EUROPEAN PATENT SPECIFICATION

(11) **EP 2 934 614 B1**
(45) Date of publication and mention of the grant of the patent: **17.03.2021**
(21) Application number: 13811556.3
(22) Date of filing: 20.12.2013
(51) Int. Cl.: A61L 27/54, A61L 27/34, A61L 27/18, A61L 27/58, A61L 29/06, A61L 29/14, A61L 29/16, A61L 31/06, A61L 31/14, A61L 31/16, C09D 177/12, C08L 77/12, C08G 69/44

(54) **COATING COMPRISING POLYESTERAMIDE COPOLYMERS FOR DRUG DELIVERY**
BESCHICHTUNG AUS POLYESTERAMID-COPOLYMEREN ZUR ARZNEIMITTELABGABE
REVÊTEMENT COMPRENANT DES COPOLYMÈRES DE POLYESTERAMIDE POUR L'ADMINISTRATION DE MÉDICAMENTS

(30) Priority: 20.12.2012 EP 12198592; 20.12.2012 US 201261740332 P
(43) Date of publication of application: 28.10.2015
(73) Proprietor: DSM IP Assets B.V., 6411 TE Heerlen (NL)
(72) Inventor: MIHOV, George, 6100 AA Echt (NL); ZUPANCICH, John Andrew, 6100 AA Echt (NL)
(74) Representative: DSM Intellectual Property
(86) International application number: PCT/EP2013/077643
(87) International publication number: WO 2014/096339

(56) References cited:
- WO-A1-2011/045443
- WO-A1-2012/175746

## Description

The present invention relates to coatings comprising a biodegradable polymer. The present invention further relates to implantable devices comprising the coating.

Coatings comprising biodegradable polymers are known in the art.

Coatings comprising biodegradable polymers such as polyesteramides are for example disclosed in WO2011045443. The disclosed biodegradable polyesteramides comprise at least two linear saturated or unsaturated aliphatic diol residues into two bis-(a amino acid)-based diol-diesters according to structural formula I,

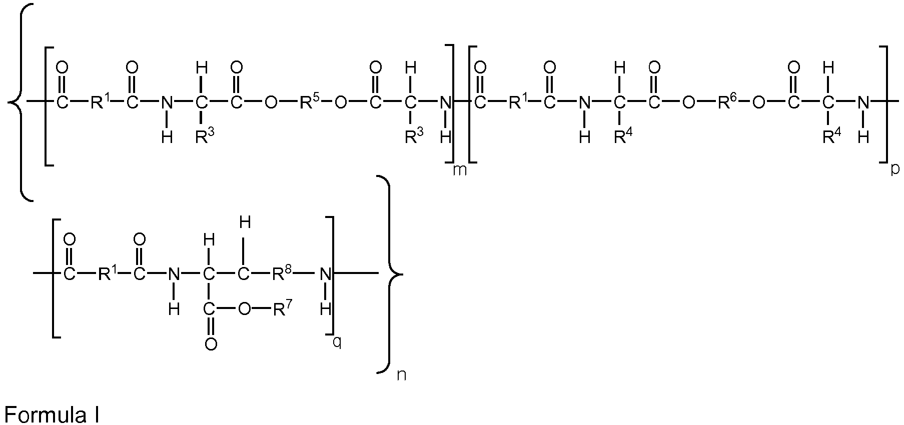

In a preferred embodiment m varies from 0.01 to 0.99; p varies from 0.2 to 3 and q varies from 0.10 to 1.00 whereby n is 5 to 100; R₁ is -(CH2)8; R₃ and R₄ in the backbone units m and p is leucine, R₅ is hexane, and R₆ is a bicyclic-fragment of 1,4:3,6-dianhydrohexitols of structural formula (II); 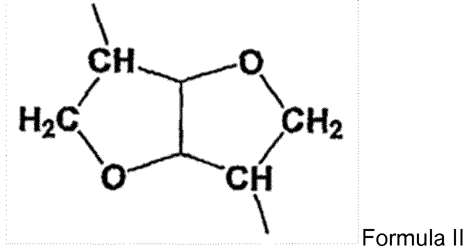 R₇ may be chosen from H or a benzyl group and R₈ is -(CH₂)4-.

If R₇ is H the polymer is further indicated as PEA-III-H, if R₇ is benzyl, the polymer is further indicated as PEA-III-Bz. However, it has been noticed that coatings based on PEA-III-H or PEA-III-Bz do not fulfill the high requirements in biomedical industry in terms of adhesion to substrates, degradation and drug release.

The high requirements in biomedical industry demand a constant improvement of the quality of polymer coatings on the medical devices. They must develop towards better adhesion to the device, to not generate particulates and when made from biodegradable polymers, they have to degrade in the targeted time period. This is particularly important for biodegradable, drug eluting coatings which should degrade completely shortly after the complete elution of the bioactive agent. There is a need for improved adhesion properties for example for coating medical devices or device components because this eliminates the need for pretreatment of the substrate or the use of a primer.

The object of the present invention is to take away the above stated drawbacks.

The object of the present invention is to provide a coating comprising a biodegradable polymer which provides an enhanced adhesion to substrates (i.e., medical devices and device components).

Another object of the present invention is to provide a coating with an increased degradation rate in the presence of enzymes.

Still another object of the present invention is to provide a coating with distinctive drug-release properties.

The object of the present invention is achieved by providing a coating having a thickness of from 1 µm to 100 µm, comprising a biodegradable polyesteramide (PEA) polymer which comprises at least two different functional groups pendant to the polymer backbone, whereby the two different functional groups comprise at least an unprotected hydrophilic functional group chosen from a carboxyl group and at least a protected hydrophobic functional group chosen from an ester group wherein the ratio of unprotected hydrophilic groups to protected hydrophobic functional groups varies from 0.17-3, and wherein the polyesteramide is a copolymer according to structural Formula (III), 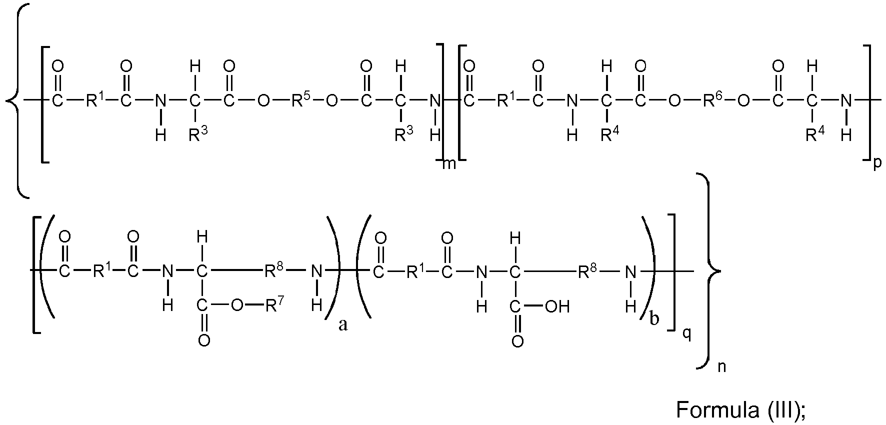
wherein m+p varies from 0.9-0.1 and q varies from 0.1 to 0.9; m+p+q=1 whereby m or p could be 0; n varies from 5 to 300 and wherein a is at least 0.1, b is at least 0.15 and a+b=1;
wherein the m unit, p unit, a unit, and b unit are each randomly distributed throughout the PEA;
whereby:
   - R₁ is independently selected from the group consisting of (C₂-C₂₀) alkyl;
   - R₃ and R₄ in a single backbone unit m or p, respectively, are independently selected from the group consisting of hydrogen, (C₁-C₆)alkyl, (C₂-C₆)alkenyl, (C₂-C₆)alkynyl, (C₆-C₁₀)aryl, -(CH₂)SH, -(CH₂)₂S(CH)₃,(CH₃)₂-CH-CH₂-, - CH(CH₃)₂, -CH(CH₃)-CH₂-CH₃, -CH₂-C₆H₅, -(CH₂)₄-NH₂, and mixtures thereof;
   - R₅ is independently selected from the group consisting of (C₂-C₂₀)alkyl, (C₂-C₂₀)alkylene;
   - R₆ is selected from bicyclic-fragments of 1,4:3,6-dianhydrohexitols of structural formula (II); 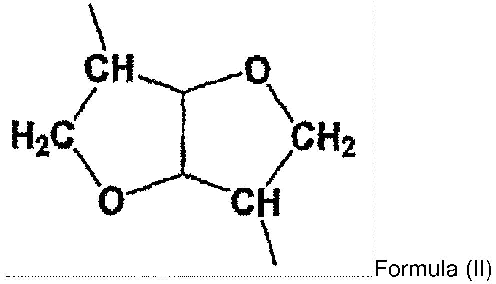
   - R₇ is independently selected from the group consisting of (C₆-C₁₀) aryl (C₁-C₆) alkyl or a protecting group; and
   - R₈ is -(CH₂)₄-.

Surprisingly it has been found that the coatings based on biodegradable polymers comprising different functional groups at a relative ratio in the polymer backbone provide enhanced adhesion to substrates (i.e., medical devices and device components). Increased adhesion the polymers on the surfaces of medical devices & device components (e.g., metal, plastic) may translate into enhanced coating properties, such as eliminating the need for pretreatment or use of a primer. Enhanced coating properties also provide improved coating homogeneity and improved wear resistance.

The coatings of the present invention moreover show an increased degradation rate of in the presence of enzymes and show distinctive drug-release properties. The results indicate an overall better performance of the coating comprising a biodegradable polymer combining two different functional groups over similar type of biodegradable polymers possessing only one the groups.

The two different functional groups comprise at least an unprotected hydrophilic functional group chosen from a carboxyl- group and at least a protected hydrophobic functional group chosen from an ester group. Unprotected means a free carboxylic group or -COOH group. By the protected hydrophobic functional group is meant a -COOR group in which R can be a (C₆-C₁₀) aryl (C₁-C₆)alkyl group.

The ratio of unprotected hydrophilic groups to protected hydrophobic functional groups varies from 0.17-3, preferably 0.3-1.

The coating according to the present invention comprises a biodegradable polymer which possesses pendant unprotected carboxyl- and protected ester functional groups.

The coating comprises a biodegradable polyesteramide (PEA).

The coating comprises a biodegradable polyesteramide copolymer (PEA) according to structural Formula (III), 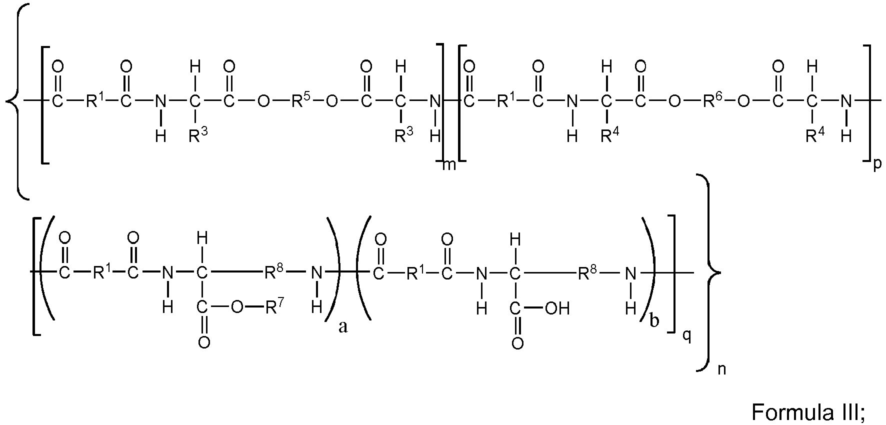
wherein m+p varies from 0.9-0.1 and q varies from 0.1 to 0.9; m+p+q=1 whereby m or p could be 0; n varies from 5 to 300 and wherein a is at least 0.1, b is at least 0.15 and a+b=1;
wherein the m unit, p unit, a unit, and b unit are each randomly distributed throughout the PEA;
and whereby:
   - R₁ is independently selected from the group consisting of (C₂-C₂₀) alkyl or (C₂-C₂₀) alkylene;
   - R₃ and R₄ in a single backbone unit m or p, respectively, are independently selected from the group consisting of hydrogen, (C₁-C₆)alkyl, (C₂-C₆)alkenyl, (C₂-C₆)alkynyl, (C₆-C₁₀aryl, -(CH₂)SH, -(CH₂)₂S(CH₃), -(CH₃)₂-CH-CH₂- , -CH(CH₃)₂, -CH(CH₃)-CH₂-CH₃,-CH₂-C₆H₅, -(CH₂)₄-NH₂, and mixtures thereof;
   - R₅ is independently selected from the group consisting of (C₂-C₂₀)alkyl, (C₂-C₂₀)alkylene;
   - R₆ is selected from bicyclic-fragments of 1,4:3,6-dianhydrohexitols of structural formula (III); 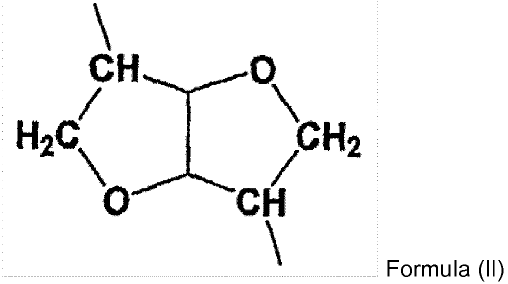
   - R₇ is independently selected from the group consisting of (C₆-C₁₀) aryl (C₁-C₆)alkyl or a protecting group; and
   - R₈ is -(CH₂)₄-.

In case that m and p are different from 0 and m+p+q=1, the PEA is referred to as PEA-III-H/Bz. The m and/or p, a and b units in the polyesteramide backbone of formula (III) are in a random distribution.

It has been found that a coating comprising a polyesteramide according to formula (III) in which a protected functional group is present in backbone unit (a) and in which an unprotected functional group is present in the backbone unit (b), whereby the percentage of unprotected functional groups in backbone unit (b) is at least 15% and the total of unprotected and protected functional groups is 100% provides improved adhesion properties to substrates such as metals, glass or plastics. Preferably the percentage of unprotected functional groups in backbone unit (b) varies in the range from 25%-75%, whereby the total of unprotected and protected functional groups is 100%.

A PEA comprising at least 15% L-lysine-COOH and thus 85% L-Lysine-COO-Benzyl (Bz) is further referred to as PEA-H/Bz 15%H. A PEA comprising at least 25% L-lysine-COOH and thus 75% L-Lysine-COO-Benzyl (Bz) is further referred to as PEA-H/Bz 25%H. A PEA comprising at least 50% L-lysine-COOH and thus 50% L-Lysine-COO-Benzyl (Bz) is further referred to as PEA-H/Bz 50%H. In case that m and p are different from 0 and m+p+q=1 and the PEA comprises 15% L-lysine-COOH and thus 85% L-Lysine-COO-Benzyl (Bz) it is further referred to as PEA-III-H/Bz 15%H, PEA-H/Bz or PEA-III-H/Bz is also further referred to in this text as PEA-X or PEA-III-X.

Furthermore, it was found that the coatings according to the present invention show an accelerated degradation after 40 days without compromising material properties and performance. The increased degradation rate of PEA- H/Bz as compared to the prior art PEA-III- Bz facilitates more rapid coating degradation and clearance/elimination from the body, which is a favorable property for numerous applications where a resorbable coating is desired.

The coatings of the present invention comprising PEA-H/Bz polymers moreover show a slow and limited swelling. This provides a means to release hydrophobic drugs over extended periods of time.

In one embodiment the coating comprises a biodegradable polyesteramide copolymer according to Formula (III) comprising p=0 and m+q=1 whereby m=0.75, a=0.5 and a+b=1 R₁ is (CH₂)₈, R₃ is (CH₃)₂-CH-CH₂-, R₅ is hexyl, R₇ is benzyl and R₈ is -(CH₂)₄-.

In another embodiment of the present invention the coating comprises a biodegradable polyesteramide copolymer according to Formula (III) comprising m+p+q=1, q=0.25, p=0.45 and m=0.3 whereby a is 0.5 and a+b=1 and whereby R₁ -(CH₂)₄-, R₃ and R₄ respectively are (CH₃)₂-CH-CH₂-, R₅ is selected from the group consisting of (C₂-C₂₀)alkylene, R₆ is selected from bicyclic-fragments of 1,4:3,6-dianhydrohexitols of structural formula (II), R₇ is benzyl, and R₈ is -(CH₂)₄-.

In a further embodiment of the present invention the coating comprises a biodegradable polyesteramide copolymer according to Formula (III) comprising m+p+q=1, q=0.25, p=0.45 and m=0.3 whereby b is 0.25 and a+b=1 whereby R₁ -(CH₂)₈-, R₃ and R₄ respectively are (CH₃)₂-CH-CH₂-, R₅ is selected from the group consisting of (C₂-C₂₀)alkylene, R₆ is selected from bicyclic-fragments of 1,4:3,6-dianhydrohexitols of structural formula (II), R₇ is benzyl, and R₈ is -(CH₂)₄-.

In a still further embodiment of the present invention the coating comprises a biodegradable polyesteramide copolymer according to Formula (III) comprising m=0, p+q=1 and p=0.75 whereby a= 0.5 and a+b=1, R₁ is-(CH₂)₄-, R₄ is (CH₃)₂-CH-CH₂-, R₇ is benzyl, R₈ is -(CH₂)₄-, and R₆ is selected from bicyclic fragments of 1,4:3,6-dianhydrohexitols of structural formula (II).

In another preferred embodiment of the present invention the coating comprises a biodegradable poly(esteramide) copolymer according to Formula (III) comprising m+p+q=1, q=0.1, p=0.30 and m=0.6 whereby a=0.5 and a+b=1. R₁ -(CH₂)₄; R₃ and R₄ respectively, are (CH₃)₂-CH-CH₂-; R₅ is selected from the group consisting of (C₂-C₂₀)alkylene, R₇ is benzyl, R₈ is -(CH₂)₄- and R₆ is selected from bicyclic-fragments of 1,4:3,6-dianhydrohexitols of structural formula (II).

In another embodiment of the present invention the coating comprises a biodegradable poly(esteramide) copolymer according to Formula (III) comprising m+p+q=1, q=0.25, p=0.45 and m=0.3 whereby b=0.5 and a+b=1. R₁ -(CH₂)₈; R₃ and R₄ respectively, are (CH₃)₂-CH-CH₂-; R₅ is selected from the group consisting of (C₂-C₂₀)alkylene, R₇ is benzyl, R₈ is -(CH₂)₄- and R₆ is selected from bicyclic-fragments of 1,4:3,6-dianhydrohexitols of structural formula (II).

At least one of the alpha-amino acids used in the polyesteramides of Formula (III) is a natural alpha-amino acid. For example, when the R₃s or R₄s are CH₂Ph, the natural alpha-amino acid used in synthesis is L-phenylalanine. In alternatives wherein the R₃s or R₄s are -CH₂-CH(CH₃)₂, the co-polymer contains the natural amino acid leucine. By independently varying the R₃s and R₄s within variations of the two co-monomers as described herein, other natural alpha -amino acids can also be used, e.g., glycine (when the R₃s or R₄s are H), alanine (when the R₃s or R₄s are CH₃), valine (when the R₃s or R₄s are CH(CH₃)₂), isoleucine (when the R₃s or R₄s are CH(CH₃)-CH₂-CH₃), phenylalanine (when the R₃s or R₄s are CH₂-C₆H₅), lysine (when the R₃s or R₄s (CH₂)₄--NH₂); or methionine (when the R₃s or R₄s are -(CH₂)₂S(CH₃), and mixtures thereof.

The polyesteramide's preferably have an average number molecular weight (Mn) ranging from 15,000 to 200,000 Daltons. The polyesteramide can be fabricated in a variety of molecular weights and a variety of relative proportions of the m, p, and q units in the backbone. The appropriate molecular weight for a particular use is readily determined by one skilled in the art. A suitable Mn will be in the order of about 15,000 to about 100,000 Daltons, for example from about 30,000 to about 80,000 or from about 35,000 to about 75,000. Mn is measured via GPC in THF with polystyrene as a standard.

The basic polymerization process of polyesteramides is based on the process described by G. Tsitlanadze, et al. J. Biomater. Sci. Polym. Edn. (2004) 15:1-24, however different building blocks and activating groups were used.

The polyesteramides of Formula (III) may for example be synthesized as shown in scheme 1; via solution polycondensation of para-toluene sulfonate diamines salts (X1, X2, X3, X4) with activated di-acids (Y1). Typically, dimethylsulfoxide or dimethylformamide are used as solvent. Generally, as a base 1.1eq triethylamide is added with respect to the amount of para-toluene sulfonate, the reaction is carried out under an inert atmosphere at 60°C for 24-72 hours under constant stirring. Subsequently the obtained reaction mixture is purified via a water precipitation followed by an organic precipitation and filtration. Drying under reduced pressure yields the polyesteramide.

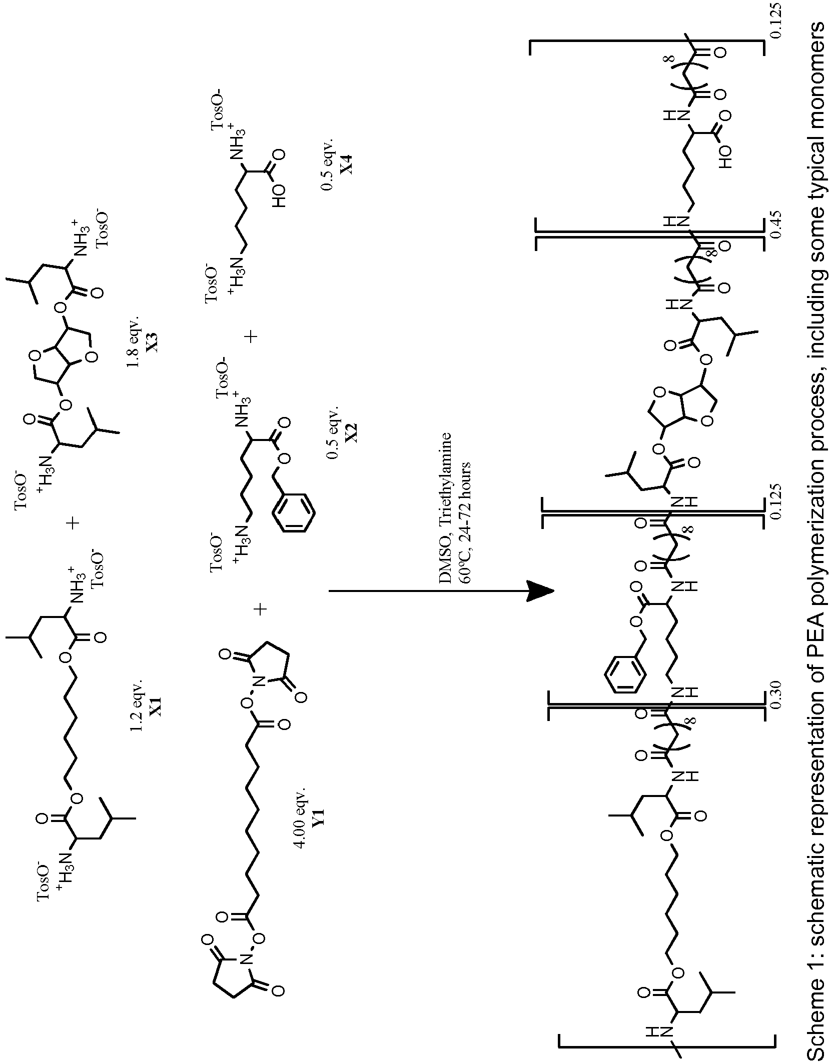

The coating according to the present invention can be a single layer coating but can also be a multilayer coating comprising more coating layers to tune the release of bioactive agents.

The coating according to the present invention has a thickness of from 1 µm to 100 µm. More preferably the coating has a thickness of about 2-75 µm, still more preferably a thickness of about 2-50 µm, most preferably a thickness of about 2-15 µm.

The term biodegradable or bioerodable as used herein refers to polymers which are capable of being completely or substantially degraded or eroded when exposed to an in vivo environment or a representative in vitro environment. A polymer is capable of being degraded or eroded when it can be gradually broken-down, resorbed, absorbed and/or eliminated by, for example, hydrolysis, hydration, solubilization, enzymolysis, oxidation, metabolic processes, bulk or surface erosion, and the like within a subject. It should be appreciated that traces or residue of polymer may remain following biodegradation and bioerosion.

The coating according to the present invention may further comprise a bioactive agent. The bioactive agent may be a therapeutic, prophylactic, or diagnostic agent. These agents can have antiproliferative or anti-inflammatory properties or can have other properties such as antineoplastic, antiplatelet, anti-coagulant, anti-fibrin, antithrombotic, antimitotic, antibiotic, antiallergic, or antioxidant properties. Moreover, these agents can be cystostatic agents, agents that promote the healing of the endothelium, or agents that promote the attachment, migration and proliferation of endothelial cells while quenching smooth muscle cell proliferation. Examples of suitable therapeutic and prophylactic agents include synthetic inorganic and organic compounds, proteins and peptides, polysaccharides and other sugars, lipids, and DNA and RNA nucleic acid sequences having therapeutic, prophylactic or diagnostic activities. Nucleic acid sequences include genes, antisense molecules, which bind to complementary DNA to inhibit transcription, and ribozymes. Some other examples of bioactive agents include antibodies, receptor ligands, enzymes, adhesion peptides, blood clotting factors, inhibitors or clot dissolving agents, such as streptokinase and tissue plasminogen activator, antigens for immunization, hormones and growth factors, oligonucleotides such as antisense oligonucleotides and ribozymes and retroviral vectors for use in gene therapy. Examples of antiproliferative agents include rapamycin and its functional or structural derivatives, 40-O-(2-hydroxy)ethyl-rapamycin (everolimus), and its functional or structural derivatives, paclitaxel and its functional and structural derivatives. Examples of rapamycin derivatives include ABT-578, 40-0-(3-hydroxy)propyl-rapamycin, 40-O-[2-(2-hydroxy)ethoxy]ethyl-rapamycin, and 40-0-tetrazole-rapamycin. Examples of paclitaxel derivatives include docetaxel. Examples of antineoplastics and/or antimitotics include methotrexate, azathioprine, vincristine, vinblastine, fluorouracil, doxorubicin hydrochloride (e.g. Adriamycin(R) from Pharmacia AND Upjohn, Peapack NJ.), and mitomycin (e.g. Mutamycin(R) from Bristol-Myers Squibb Co., Stamford, Conn.). Examples of such antiplatelets, anticoagulants, antifibrin, and antithrombins include sodium heparin, low molecular weight heparins, heparinoids, hirudin, argatroban, forskolin, vapiprost, prostacyclin and prostacyclin analogues, dextran, D-phe-pro-arg-chloromethylketone (synthetic antithrombin), dipyridamole, glycoprotein Hb/nia platelet membrane receptor antagonist antibody, recombinant hirudin, thrombin inhibitors such as Angiomax (Biogen, Inc., Cambridge, Mass.), calcium channel blockers (such as nifedipine), colchicine, fibroblast growth factor (FGF) antagonists, fish oil (omega 3-fatty acid), histamine antagonists, lovastatin (an inhibitor of HMG-CoA reductase, a cholesterol lowering drug, brand name Mevacor(R) from Merck and Co., Inc., Whitehouse Station, NJ), monoclonal antibodies (such as those specific for Platelet-Derived Growth Factor (PDGF) receptors), nitroprusside, phosphodiesterase inhibitors, prostaglandin inhibitors, suramin, serotonin blockers, steroids, thioprotease inhibitors, triazolopyrimidine (a PDGF antagonist), super oxide dismutases, super oxide dismutase mimetic, 4-amino-2,2,6,6-tetramethylpiperidine-I-oxyl (4-amino-TEMPO), estradiol, anticancer agents, dietary supplements such as various vitamins, and a combination thereof. Examples of anti-inflammatory agents including steroidal and nonsteroidal anti-inflammatory agents include biolimus, tacrolimus, dexamethasone, clobetasol, corticosteroids or combinations thereof. Examples of such cytostatic substances include angiopeptin, angiotensin converting enzyme inhibitors such as captopril (e.g. Capoten(R) and Capozide(R) from Bristol-Myers Squibb Co., Stamford, Conn.), cilazapril or lisinopril (e.g. Prinivil(R) and Prinzide(R) from Merck and Co., Inc., Whitehouse Station, NJ). An example of an antiallergic agent is permirolast potassium. Other therapeutic substances or agents which may be appropriate include alpha-interferon, pimecrolimus, imatinib mesylate, midostaurin, and genetically engineered epithelial cells. The foregoing substances can also be used in the form of prodrugs or co-drugs thereof. The foregoing substances also include metabolites thereof and/or prodrugs of the metabolites. The foregoing substances are listed by way of example and are not meant to be limiting.

The coating according to the present invention may comprise more than one biooactive agent. A further bioactive agent can also be chosen from the above list of bioactive agents.

The coating according to the present invention may comprise the bioactive agent in a dispersed or dissolved form. The bioactive agent may be present the form of microparticles, nanoparticles or micelles.

In a further embodiment the coating according to the present invention may comprise a blend of different PEA polymers of Formula (III) but may also comprise at least another polymer. Representative other polymers include, but are not limited to, polyhydroxyalkanoates (PHA), poly(3-hydroxyalkanoates) such as poly(3-hydroxypropanoate), poly(3-hydroxybutyrate), poly(3-hydroxyvalerate), poly(3-hydroxyhexanoate), poly(3-hydroxyheptanoate) and poly(3-hydroxyoctanoate), poly(4-hydroxyalkanaote) such as poly(4-hydroxybutyrate), poly(4-hydroxyvalerate), poly(4-hydroxyhexanote), poly(4-hydroxyheptanoate), poly(4-hydroxyoctanoate) and copolymers including any of the 3-hydroxyalkanoate or 4-hydroxyalkanoate monomers described herein or blends thereof, poly(D,L-lactide), poly(L-lactide), polyglycolide, poly(D,L-lactide-co- glycolide), poly(L-lactide-co-glycolide), polycaprolactone, poly(lactide-co-caprolactone), poly(glycolide-co-caprolactone), poly(dioxanone), poly(ortho esters), poly(trimethylene carbonate), poly(anhydrides), poly(tyrosine carbonates) and derivatives thereof, poly(tyrosine ester) and derivatives thereof, poly(imino carbonates), poly(glycolic acid-co-trimethylene carbonate), polyphosphoester, polyphosphoester urethane, poly(amino acids), polycyanoacrylates, poly(iminocarbonate), polyurethanes, polyoxymethylenes, polyimides, polyethers, poly(glyceryl sebacate), poly(propylene fumarate), poly(n-butyl methacrylate), poly(sec-butyl methacrylate), poly(isobutyl methacrylate), poly(tert-butyl methacrylate), poly(n-propyl methacrylate), poly(isopropyl methacrylate), poly(ethyl methacrylate), poly(methyl methacrylate), polyurethanes, rayon, rayon- triacetate, cellulose acetate, cellulose butyrate, cellulose acetate butyrate, cellophane, cellulose nitrate, cellulose propionate, cellulose ethers, carboxymethyl cellulose, polyethers such as poly(ethylene glycol) (PEG), copoly(ether-esters) (e.g. PEO/PLA), polyalkylene oxides such as poly(ethylene oxide), poly(propylene oxide), poly(ether ester), polyalkylene oxalates, polyphosphazenes, phosphoryl choline, choline, poly(aspirin), polymers and copolymers of hydroxyl bearing monomers such as HEMA, hydroxypropyl methacrylate (HPMA), hydroxypropylmethacrylamide, PEG acrylate (PEGA), PEG methacrylate, 2-methacryloyloxyethylphosphorylcholine (MPC) and n-vinyl pyrrolidone (VP), carboxylic acid bearing monomers such as methacrylic acid (MA), acrylic acid (AA), alkoxymethacrylate, alkoxyacrylate, and 3-trimethylsilylpropyl methacrylate (TMSPMA), poly(styrene-isoprene-styrene)-PEG (SIS-PEG), polystyrene-PEG, polyisobutylene-PEG, polycaprolactone-PEG (PCL-PEG), PLA-PEG, poly(methyl methacrylate)-PEG (PMMA- PEG), polydimethylsiloxane-co-PEG (PDMS-PEG), poly(vinylidene fluoride)-PEG (PVDF- PEG), PLURONIC™ surfactants (polypropylene oxide-co-polyethylene glycol), poly(tetramethylene glycol), hydroxy functional poly(vinyl pyrrolidone), biomolecules such as collagen, chitosan, alginate, fibrin, fibrinogen, cellulose, starch, collagen, dextran, dextrin, fragments and derivatives of hyaluronic acid, heparin, fragments and derivatives of heparin, glycosamino glycan (GAG), GAG derivatives, polysaccharide, elastin, chitosan, alginate, or combinations thereof.

In a still further embodiment, the coating may comprise a biobeneficial material. The biobeneficial material can be polymeric or non-polymeric. The biobeneficial material is preferably substantially non-toxic, non-antigenic and non-immunogenic. A biobeneficial material preferably enhances the biocompatibility of the coating by being non-fouling, hemocompatible, actively non-thrombogenic, or anti-inflammatory, all without depending on the release of the bioactive agent.

Representative biobeneficial materials include, but are not limited to, polyethers such as poly(ethylene glycol), copoly(ether-esters) (e.g. PEO/PLA), polyalkylene oxides such as poly(ethylene oxide), poly(propylene oxide), poly(ether ester), polyalkylene oxalates, polyphosphazenes, phosphoryl choline, choline, poly(aspirin), polymers and co-polymers of hydroxyl bearing monomers such as hydroxyethyl methacrylate (HEMA), hydroxypropyl methacrylate (HPMA), hydroxypropylmethacrylamide, poly (ethylene glycol) acrylate (PEGA), PEG methacrylate, 2-methacryloyloxyethylphosphorylcholine (MPC) and «-vinyl pyrrolidone (VP), carboxylic acid bearing monomers such as methacrylic acid (MA), acrylic acid (AA), alkoxymethacrylate, alkoxyacrylate, and 3-trimethylsilylpropyl methacrylate (TMSPMA), poly(styrene-isoprene-styrene)-PEG (SIS-PEG), polystyrene-PEG, polyisobutylene-PEG, polycaprolactone-PEG (PCL-PEG), PLA-PEG, poly(methyl methacrylate)-PEG (PMMA-PEG), polydimethylsiloxane-co-PEG (PDMS-PEG), poly(vinylidene fluoride)-PEG (PVDF-PEG), Pluronic™ surfactants (polypropylene oxide-co-polyethylene glycol), poly(tetramethylene glycol), hydroxy functional poly(vinyl pyrrolidone), biomolecules such as fibrin, fibrinogen, cellulose, starch, collagen, dextran, dextrin, hyaluronic acid, fragments and derivatives of hyaluronic acid, heparin, fragments and derivatives of heparin, glycosamino glycan (GAG), GAG derivatives, polysaccharide, elastin, chitosan, alginate, silicones, PolyActive™, or combinations thereof. In some embodiments, the coating can exclude any one of the aforementioned polymers. The term PolyActive™ refers to a block copolymer having flexible poly(ethylene glycol) and poly(butylene terephthalate) blocks (PEGTVPBT). PolyActive™ is intended to include AB, ABA, BAB copolymers having such segments of PEG and PBT (e.g., poly(ethylene glycol)-block-poly(butyleneterephthalate)-block poly(ethylene glycol) (PEG- PBT-PEG).

The present invention further relates to an implantable device comprising the coating. The implantable device herein can be used to treat, prevent, or ameliorate a medical condition such as atherosclerosis, thrombosis, restenosis, hemorrhage, vascular dissection or perforation, vascular aneurysm, vulnerable plaque, chronic total occlusion, claudication, anastomotic proliferation (for vein and artificial grafts), bile duct.

As used herein, an implantable device may be any suitable medical substrate that can be implanted in a human or veterinary patient. Examples of such medical devices include self- expandable stents, balloon-expandable stents, stent-grafts, grafts (e.g., aortic grafts), heart valve prostheses, cerebrospinal fluid shunts, pacemaker electrodes, catheters, and endocardial leads (e.g., FINELINE and ENDOTAK, available from Guidant Corporation, Santa Clara, CA), anastomotic devices and connectors, orthopedic implants such as screws, spinal implants, and electro-stimulatory devices. The underlying structure of the device can be of virtually any design. The substrate can be made of a metallic material or an alloy such as, but not limited to, cobalt chromium alloy (ELGILOY), stainless steel (316L), high nitrogen stainless steel, e.g., BIODUR 108, cobalt chrome alloy L-605, "MP35N," "MP20N," ELASTINITE (Nitinol), tantalum, nickel-titanium alloy, platinum-indium alloy, gold, magnesium, or combinations thereof. "MP35N" and "MP20N" are trade names for alloys of cobalt, nickel, chromium and molybdenum available from Standard Press Steel Co., Jenkintown, PA. "MP35N" consists of 35 percent cobalt, 35 percent nickel, 20 percent chromium, and 10 percent molybdenum. "MP20N" consists of 50 percent cobalt, 20 percent nickel, 20 percent chromium, and 10 percent molybdenum. Devices made from bioabsorbable (e.g., bioabsorbable stent) or biostable polymers could also be used with the embodiments of the present invention.

Preferably, the implantable device is a stent. The stent described herein is useful for a variety of medical procedures, including, by way of example, treatment of obstructions caused by tumors in bile ducts, esophagus, trachea/bronchi and other biological passageways. A stent having the above-described coating is particularly useful for treating diseased regions of blood vessels caused by lipid deposition, monocyte or macrophage infiltration, or dysfunctional endothelium or a combination thereof, or occluded regions of blood vessels caused by abnormal or inappropriate migration and proliferation of smooth muscle cells, thrombosis, and restenosis. Stents may be placed in a wide array of blood vessels, both arteries and veins. Representative examples of sites include the iliac, renal, carotid and coronary arteries.

The coating according to the present invention can be applied to the substrate in many ways, such as dip-coating, spray-coating, ionic deposition, and the like. These coating processes are well known in the art. Preferably the coating of the present invention is spray coated on the substrate.

The dosage or concentration of the bioactive agent required to produce a favorable therapeutic effect should be less than the level at which the bioactive agent produces toxic effects and greater than the level at which non-therapeutic results are obtained. The dosage or concentration of the bioactive agent can depend upon factors such as the particular circumstances of the patient, the nature of the trauma, the nature of the therapy desired, the time over which the ingredient administered resides at the vascular site, and if other active agents are employed, the nature and type of the substance or combination of substances. Therapeutically effective dosages can be determined empirically, for example by infusing vessels from suitable animal model systems and using immuno-histochemical, fluorescent or electron microscopy methods to detect the agent and its effects, or by conducting suitable in vitro studies. Standard pharmacological test procedures to determine dosages are understood by those of ordinary skill in the art.

The present invention will now be described in detail with reference to the following non-limiting examples which are by way of illustration only.

### EXAMPLES

### Example 1

PEA discs were generated via compression molding of cryogenically milled polymer powders. Using a circular mold, PEA powder was loaded into the mold and discs (diameter = 25 mm; thickness = 1 mm) were formed using the following conditions:
5 minutes at T=140°C, pressure (P) 0kN, 3 minutes at T=140°C, P=10kN, 3 minutes at T=140°C, P=50kN at a pressure of 180kN while cooling to T=25°C.

To prevent adhesion of the material to the compression plates Teflon foil was used.

The PEA discs were then placed in the middle of 30 mm diameter metal compression plates (stainless steel - D15070; GABO Qualimeter Testanlangen GmbH; Ahlden, Germany) and the polymer samples mounted to the plates using the following conditions: 10 minutes at T=140°C at a pressure of 2N while cooling to T=20°C.

As the PEA samples were heated above their melting temperature, the diameter and thickness of the discs changed. The dimensions of the PEA discs after mounting to the metal compression plates are given in Table 1.

**Table 1: Dimensions of prepared PEA discs:**

| Sample | Diameter (mm) | Thickness (mm) |
|---|---|---|
| PEA-III-Bz | 27.15 | 0.90 |
| PEA-III-25 H/Bz | 29.90 | 0.76 |
| PEA-III-50 H/Bz | 25.34 | 0.99 |
| PEA-III-75 H/Bz | 26.17 | 1.03 |
| PEA-III-H | 32.65 | 0.53 |

### Sample Testing

The compression plates (with PEA samples mounted between them) where positioned in a universal testing machine (Zwick Z010; Zwick Roell AG; Ulm, Germany) and the plates were pulled apart at 20 °C with a test speed of 0.1 mm/min until the samples ruptured (i.e., adhesive failure was observed).

### Results

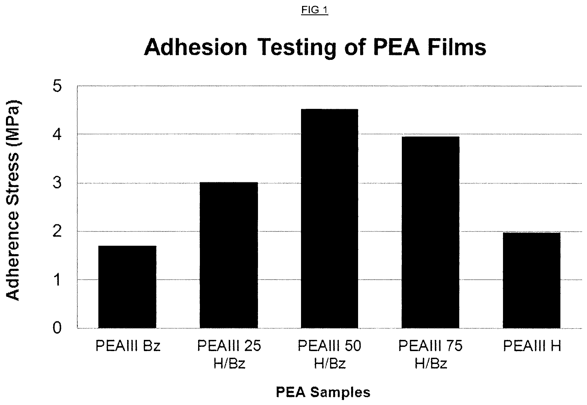

The measured force necessary to separate the compression plates between which the PEA discs were mounted as given in Table 2. The PEA III 25H/Bz, 50 H/Bz, and 75 H/Bz samples all required over 2x the force (N) to separate the plates as compared to the PEA III Bz sample. When accounting for the slight differences in sample diameter, the calculated adherence stress values for all PEA III H/Bz samples were considerably higher (>50%) than that of PEA III Bz and PEA III H samples. Figure 1. depicts the measured adherence stress for all polymer samples tested ordered from left to right on the basis of increasing H-content.

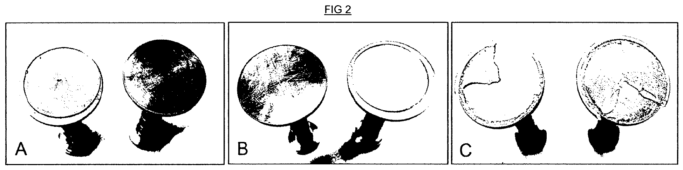

It was observed that PEA III Bz (Figure 2A) and PEA III H/Bz samples (Figure 2B; PEA III 50 H/Bz representative example) detached from the compression plates leaving little to no residue on the plate where the interface failed. Figure 2C shows that when pulled apart, the PEA III H sample failed in brittle manner with fractures observed in the bulk of the material and polymer residue left on the surface of both stainless steel plates.

**Table 2: Adhesion Testing Values**

| Sample | Sample Surface Area (mm²) | Force at break (N) | Adherence stress (MPa) |
|---|---|---|---|
| PEA-III-Bz | 578.9 | 987 | 1.70 |
| PEA-III-25 H/Bz | 702.2 | 2120 | 3.02 |
| PEA-III-50 H/Bz | 504.3 | 2290 | 4.54 |
| PEA-III-75 H/Bz | 537.9 | 2130 | 3.96 |
| PEA-III-H | 706.9 | 1400 | 1.98 |

### Example 2

Films of PEA-III-Ac Bz, PEA-III-X25, PEA-III-X50 and PEA-III-X75 were prepared by melt pressing (3 min, 120 °C, 5 bar) on stainless steel supports. The obtained films were cut into pieces of 0.5 cm² on the stainless steel supports and were individually weighted on a microbalance. The single films were immersed in 2ml PBS buffer containing 1.7U/mL α-chymotrypsin (bovine) and 0.05% sodium azide as a biocide, whereby the buffers were refreshed twice a week.

Enzymatic degradation was performed under gentle orbital shaking at 37 °C. Samples were taken in triplicate; the films were dried under reduced pressure at 45 °C overnight. The weight of the films post degradation was again determined with a microbalance.

Relative molecular weights of the remaining polymer films were determined using a Waters GPC system consisting of a Waters RI detector type 2414, a Waters separation module with column heater type e2695. The system was equipped with a Styragel HR5E and Styragel HR2 column run at 50 °C. Tetrahydrofuran (THF) was used as mobile phase with a flow rate of 1.0mL/min. Samples were dissolved in THF, of which 100µL was injected onto the column. Evaluation of data was performed with Waters_Empower² software. Calculations of molecular weights were relatively to polystyrene standards.

### FIGURES

- Figure 1:: Experimentally determined adherence stress for thin films of PEA pressed between two stainless steel plates
- Figure 2:: PEA samples after adhesion testing (A) PEA-III-Bz; (B) PEA-III-50 H/Bz; (C) PEA-III-H

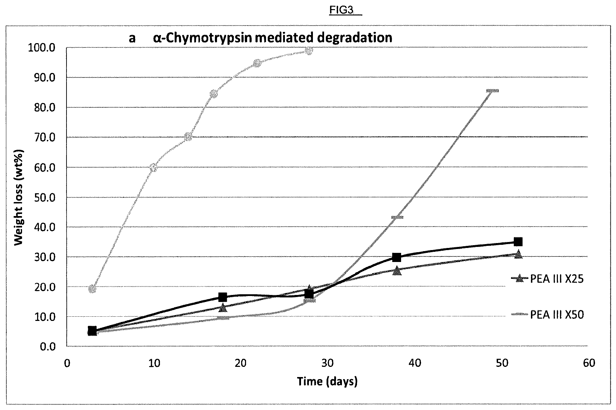

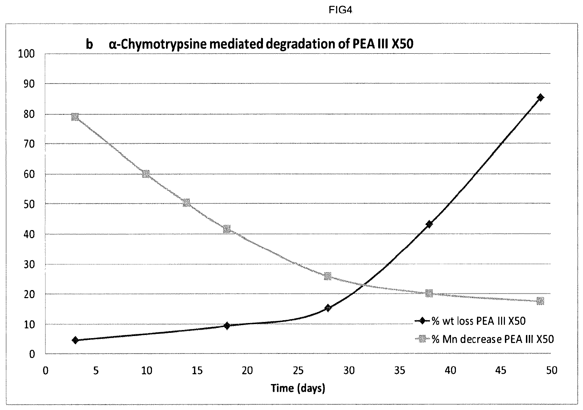

- Figure 3-4:: The polymer weight loss after immersion in a buffer with 1.7 U/mL α-chymotrypsin is illustrated up to 52 days. The degradation profiles of PEA-III-25X, PEA-III-50X and PEA-III-AcBz are comparable up to 28 days, after which PEA III 50X approached the dissolution molecular weight and shows rapid degradation (Figure 4). PEA-III-75X shows very fast degradation compared to the other polymers and degrades within 28 days.

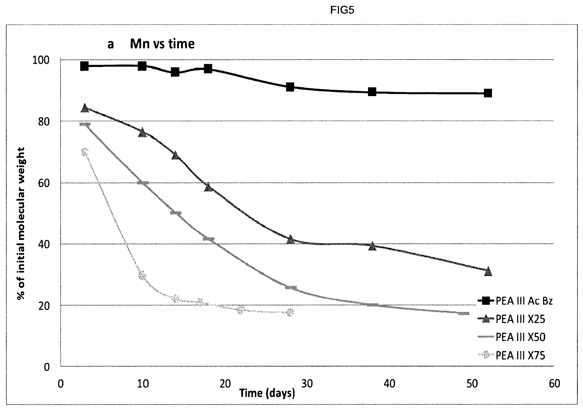

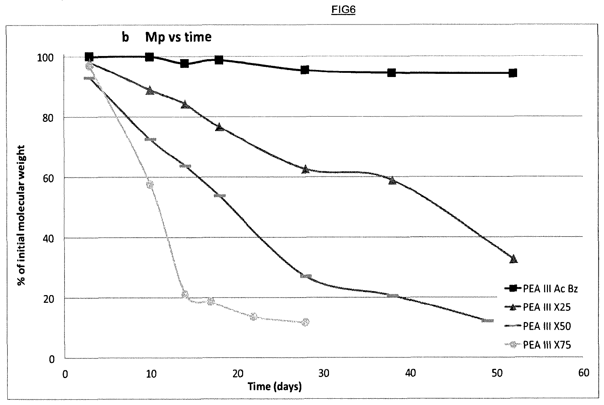

- Figure 5-6:: The relative molecular weight of the samples, immersed in PBS buffer containing 1.7U/mL α-chymotrypsin (bovine) and 0.05% sodium azide, is illustrated up to 52 days of incubation. The relative molecular weight of PEA-III-AcBz shows a slow decrease compared to the polymers which contain an increased number of carboxylic groups. These polymers show a faster molecular weight drop, illustrating that random chain scission occurs faster.

## Claims

1. Coating having a thickness of from 1 µm to 100 µm, comprising a biodegradable polyesteramide (PEA) polymer which comprises at least two different functional groups pendant to the polymer backbone, whereby the two different functional groups comprise at least an unprotected hydrophilic functional group chosen from a carboxyl group and at least a protected hydrophobic functional group chosen from an ester group wherein the relative ratio of unprotected hydrophilic groups to protected hydrophobic functional groups varies from 0.17-3, wherein the polyesteramide is a copolymer according to structural Formula (III), 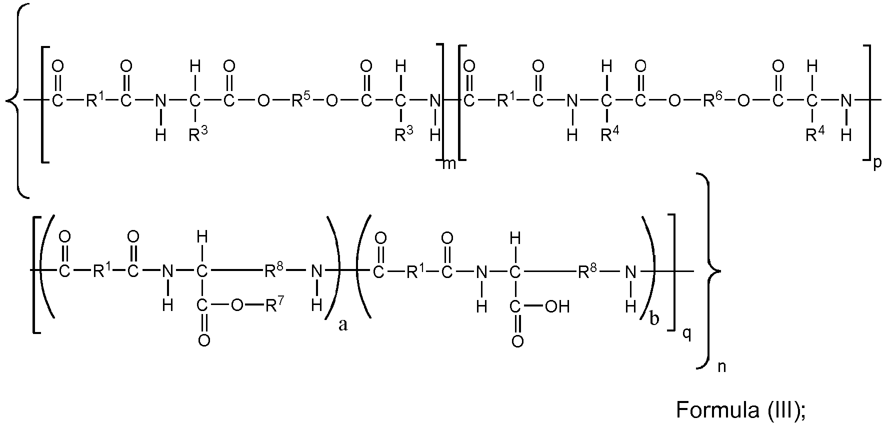
wherein m+p varies from 0.9-0.1 and q varies from 0.1 to 0.9; m+p+q=1 whereby m or p could be 0; n varies from 5 to 300 and wherein a is at least 0.1, b is at least 0.15 and a+b=1;
wherein the m unit, p unit, a unit, and b unit are each randomly distributed throughout the PEA;
whereby:
- R₁ is independently selected from the group consisting of (C₂-C₂₀) alkyl;
- R₃ and R₄ in a single backbone unit m or p, respectively, are independently selected from the group consisting of hydrogen, (C₁-C₆)alkyl, (C₂-C₆)alkenyl, (C₂-C₆)alkynyl, (C₆-C₁₀)aryl, -(CH₂)SH, -(CH₂)₂S(CH)₃,(CH₃)₂-CH-CH₂-,-CH(CH₃)₂, -CH(CH₃)-CH₂-CH₃, -CH₂-C₆H₅, -(CH₂)₄-NH₂, and mixtures thereof;
- R₅ is independently selected from the group consisting of (C₂-C₂₀)alkyl, (C₂-C₂₀)alkylene;
- R₆ is selected from bicyclic-fragments of 1,4:3,6-dianhydrohexitols of structural formula (II); 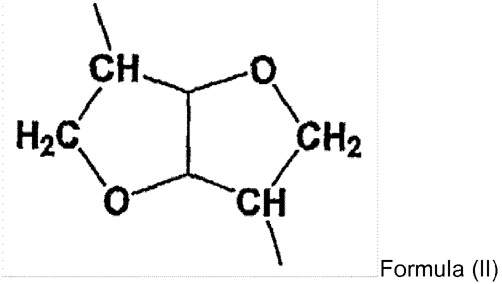
- R₇ is independently selected from the group consisting of (C₆-C₁₀) aryl (C₁-C₆) alkyl or a protecting group; and
- R₈ is -(CH₂)₄-.

2. Coating according to claim 1 in which b is at least 0.25.

3. Coating according to any one of the claims 1-2 in which b is at least 0.5.

4. Coating according to claim 1 in which b is in the range from 0.25-0.75.

5. Coating according to claim 1 wherein p=0 and m+q=1, m= 0.75, b is 0.5 and a+b=1 whereby R₁ is (CH₂)₈, R₃ is (CH₃)₂-CH-CH₂-, R₅ is hexyl, R₇ is benzyl, R₈ is -(CH₂)₄-.

6. Coating according to claim 1 wherein m+p+q=1, q=0.25, p=0.45 and m=0.3, b is 0.25 and a+b=1 whereby R₁ -(CH₂)₈-; R₃ and R₄ respectively, are (CH₃)₂-CH-CH₂-; R₅ is (C₂-C₂₀)alkylene; R₇ is benzyl; R₈ is -(CH₂)₄-; and R₆ is selected from bicyclic-fragments of 1,4:3,6-dianhydrohexitols of structural formula (II).

7. Coating according to claim 1 wherein m+p+q=1, q=0.25, p=0.45 and m=0.3, b is 0.5, a+b=1 whereby R₁ is-(CH₂)₈-; R₄ is (CH₃)₂-CH-CH₂-; R₇ is benzyl; R₈ is -(CH₂)₄-; and R₆ is selected from bicyclic-fragments of 1,4:3,6-dianhydrohexitols of structural formula (II).

8. Coating according to claim 1 wherein m+p+q=1, q=0.1, p=0.30 and m=0.6, b is 0.5 and a+b=1 whereby R₁ -(CH₂)₄-; R₃ and R₄ respectively, are (CH₃)₂-CH-CH₂-; R₇ benzyl; R₈ is -(CH₂)₄-; R₅ is selected from the group consisting of (C₂-C₂₀)alkylene; and R₆ is selected from bicyclic-fragments of 1,4:3,6-dianhydrohexitols of structural formula (II).

9. Coating according to any one of the claims 1-8 further comprising a bioactive agent selected from the group of growth factors, antibiotics, anti-inflammatory compounds, antithrombogenic compounds, anti-claudication drugs, anti-arrhythmic drugs, anti-atherosclerotic drugs, antihistamines, cancer drugs, vascular drugs, ophthalmic drugs, amino acids, vitamins, hormones, neurotransmitters, neurohormones, enzymes, imaging agents, signaling molecules and psychoactive medicaments.

10. Implantable device comprising the coating according to any one of the claims 1-9.

11. Implantable device according to claim 10, wherein the device includes cardiac pacemakers and defibrillators; leads and electrodes for the preceding, organ stimulators such as nerve, bladder, sphincter and diaphragm stimulators, prostheses, rods, vascular grafts, self-expandable stents, balloon- expandable stents, stent-grafts, grafts, catheters, artificial heart valves and cerebrospinal fluid shunts.

12. Coating according to claim 1, wherein the ratio of unprotected hydrophilic groups to protected hydrophobic functional groups varies from 0.3-1.

13. Coating according to claim 1, wherein the thickness is 2-15 µm.

## Patentansprüche

1. Überzug mit einer Dicke von 1 µm bis 100 µm, umfassend ein biologisch abbaubares Polyesteramid(PEA)-Polymer, das mindestens zwei verschiedene funktionelle Gruppen umfasst, die zur Polymerhauptkette seitenständig sind, wobei die zwei verschiedenen funktionellen Gruppen mindestens eine ungeschützte hydrophile funktionelle Gruppe, die aus einer Carboxylgruppe ausgewählt ist, und mindestens eine geschützte hydrophobe funktionelle Gruppe, die aus einer Estergruppe ausgewählt ist, umfassen, wobei das relative Verhältnis von ungeschützten hydrophilen Gruppen zu geschützten hydrophoben funktionellen Gruppen von 0,17-3 variiert, wobei es sich bei dem Polyesteramid um ein Copolymer gemäß Strukturformel (III) handelt: 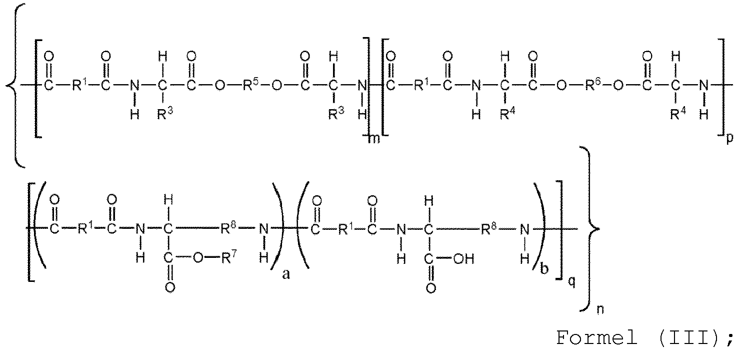
wobei m + p von 0,9-0,1 variiert und q von 0,1 bis 0,9 variiert; m + p + q = 1;
wobei m oder p 0 sein könnten; n im Bereich von 5 bis 300 variiert und wobei a mindestens 0,1 beträgt, b mindestens 0,15 beträgt und a + b = 1;
wobei die m-Einheit, p-Einheit, a-Einheit und b-Einheit jeweils statistisch über das PEA verteilt sind;
wobei:
- R₁ unabhängig aus der Gruppe bestehend aus (C₂-C₂₀)-Alkyl ausgewählt ist;
- R₃ und R₄ in einer einzelnen Hauptketteneinheit m bzw. p jeweils unabhängig aus der Gruppe bestehend aus Wasserstoff, (C₁-C₆)-Alkyl, (C₂-C₆)-Alkenyl, (C₂-C₆) -Alkinyl, (C₆-C₁₀) -Aryl, -(CH₂)SH, - (CH₂)₂S(CH)₃, (CH₃)₂-CH-CH₂-, -CH(CH₃)₂, -CH(CH₃)-CH₂-CH₃, -CH₂-C₆H₅,
- (CH₂)₄-NH₂ und Mischungen davon ausgewählt sind;
- R₅ aus der Gruppe bestehend aus (C₂-C₂₀)-Alkyl, (C₂-C₂₀)-Alkylen ausgewählt ist;
- R₆ aus bicyclischen Fragmenten von 1,4:3,6-Dianhydrohexitolen der Strukturformel (II) ausgewählt ist; 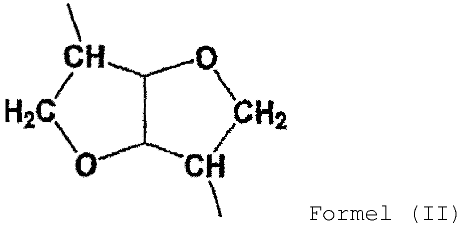
- R₇ unabhängig aus der Gruppe bestehend aus (C₆-C₁₀)-Aryl-(C₁-C₆)-alkyl oder einer Schutzgruppe ausgewählt ist und
- R₈ für -(CH₂)₄- steht.

2. Überzug nach Anspruch 1, wobei b für mindestens 0,25 steht.

3. Überzug nach einem der Ansprüche 1-2, wobei b für mindestens 0,5 steht.

4. Überzug nach Anspruch 1, wobei b im Bereich von 0,25-0,75 liegt.

5. Überzug nach Anspruch 1, wobei p = 0 und m + q = 1, m = 0,75, b für 0,5 steht und a + b = 1, wobei R₁ für (CH₂)₈ steht, R₃ für (CH₃)₂-CH-CH₂- steht, R₅ für Hexyl steht, R₇ für Benzyl steht und R₈ für -(CH₂)₄- steht.

6. Überzug nach Anspruch 1, wobei m + p + q = 1, q = 0,25, p = 0,45 und m = 0,3, b für 0,25 steht und a + b = 1, wobei R₁ für -(CH₂)₈- steht; R₃ und R₄ jeweils für (CH₃)₂-CH-CH₂- stehen; R₅ für (C₂-C₂₀) -Alkylen steht; R₇ für Benzyl steht; R₈ für -(CH₂)₄- steht und R₆ aus bicyclischen Fragmenten von 1,4:3,6-Dianhydrohexitolen der Strukturformel (II) ausgewählt ist.

7. Überzug nach Anspruch 1, wobei m + p + q = 1, q = 0,25, p = 0,45 und m = 0,3, b für 0,5 steht, a + b = 1, wobei R₁ für -(CH₂)₈- steht, R₄ für (CH₃)₂-CH-CH₂- steht; R₇ für Benzyl steht; R₈ für -(CH₂)₄- steht und R₆ aus bicyclischen Fragmenten von 1,4:3,6-Dianhydrohexitolen der Strukturformel (II) ausgewählt ist.

8. Überzug nach Anspruch 1, wobei m + p + q = 1, q = 0,1, p = 0,30 und m = 0,6, b für 0,5 steht und a + b = 1, wobei R₁ für -(CH₂)₄- steht; R₃ und R₄ jeweils für (CH₃)₂-CH-CH₂- stehen; R₇ für Benzyl steht; R₈ für -(CH₂)₄- steht; R₅ aus der Gruppe bestehend aus (C₂-C₂₀)-Alkylen ausgewählt ist und R₆ aus bicyclischen Fragmenten von 1,4:3,6-Dianhydrohexitolen der Strukturformel (II) ausgewählt ist.

9. Überzug nach einem der Ansprüche 1-8, ferner umfassend ein bioaktives Mittel aus der Gruppe der Wachstumsfaktoren, Antibiotika, entzündungshemmenden Verbindungen, antithrombogenen Verbindungen, Arzneistoffe gegen Claudicatio, Arzneistoffe gegen Arrhythmie, Arzneistoffe gegen Atherosklerose, Antihistaminika, Arzneistoffe gegen Krebs, vaskulären Arzneistoffe, ophthalmischen Arzneistoffe, Aminosäuren, Vitamine, Hormone, Neurotransmitter, Neurohormone, Enzyme, Abbildungsmittel, Signalgebungsmoleküle und psychoaktiven Medikamente.

10. Implantierbare Vorrichtung, umfassend den Überzug nach einem der Ansprüche 1-9.

11. Implantierbare Vorrichtung nach Anspruch 10, wobei die Vorrichtung Herzschrittmacher und Defibrillatoren, Leitungen und Elektroden dafür, Organstimulatoren wie Nerven-, Blasen-, Schließmuskel- und Diaphragmastabilisatoren, Prothesen, Rods, Gefäßimplantate, selbstexpandierbare Stents, ballonexpandierbare Stents, Stentgrafts, Grafts, Katheter, künstliche Herzklappen und Liquor-Shunts einschließt.

12. Überzug nach Anspruch 1, wobei das Verhältnis von ungeschützten hydrophilen Gruppen zu geschützten hydrophoben Gruppen von 0,3-1 variiert.

13. Überzug nach Anspruch 1, wobei die Dicke 2-15 µm beträgt.

## Revendications

1. Revêtement possédant une épaisseur allant de 1 µm à 100 µm, comprenant un polymère de type polyesteramide (PEA) biodégradable qui comprend au moins deux groupes fonctionnels différents pendants au squelette de polymère, les deux groupes fonctionnels différents comprenant au moins un groupe fonctionnel hydrophile non protégé choisi parmi un groupe carboxyle et au moins un groupe fonctionnel hydrophobe protégé choisi parmi un groupe ester, le rapport relatif des groupes hydrophile non protégé sur les groupes fonctionnels hydrophobes protégés variant de 0,17 à 3, le polyesteramide étant un copolymère selon la Formule structurale (III), 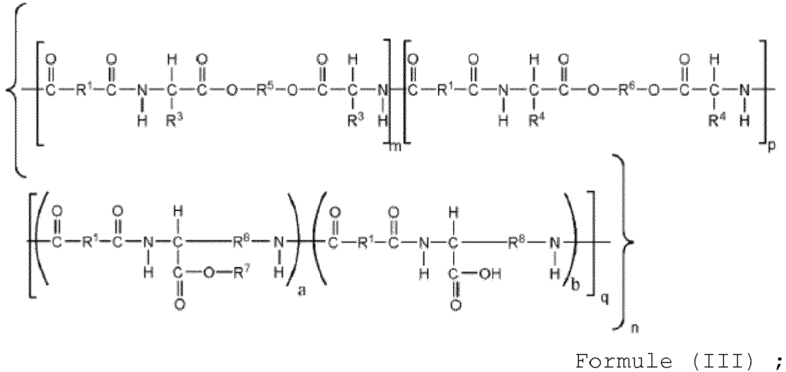
m + p variant de 0,9 à 0,1 et q variant de 0,1 à 0,9 ; m + p + q = 1, où m ou p peut être 0 ; n variant de 5 à 300 et a étant au moins 0,1, b étant au moins 0,15 et a + b = 1 ;
le motif m, le motif p, le motif a, et le motif b étant chacun distribués de manière aléatoire dans tout le PEA ;
- R₁ étant indépendamment choisi dans le groupe constitué par C₂₋₂₀-alkyle ;
- R₃ et R₄ dans un unique motif de squelette respectivement m ou p, étant indépendamment choisis dans le groupe constitué par hydrogène, C₁₋₆-alkyle, C₂₋₆-alcényle, C₂₋₆-alcynyle, C₆₋₁₀-aryle, -(CH₂)SH, - (CH₂)₂S(CH)₃, (CH₃)₂-CH-CH₂-, - CH(CH₃)₂, -CH(CH₃)-CH₂-CH₃, -CH₂-C₆H₅, - (CH₂)₄-NH₂ et des mélanges correspondants ;
- R₅ étant indépendamment choisi dans le groupe constitué par C₂₋₂₀-alkyle, C₂₋₂₀-alkylène ;
- R₆ étant choisi parmi des fragments bicycliques de 1,4:3,6-dianhydrohexitols de formule structurale (II) ; 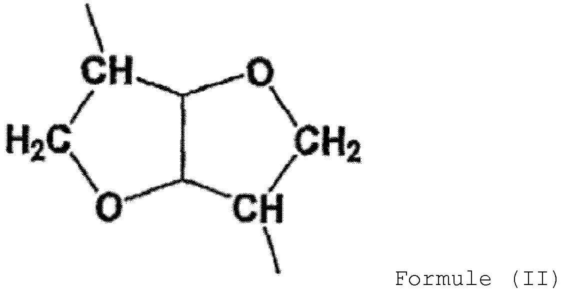
- R₇ étant indépendamment choisi dans le groupe constitué par C₆₋₁₀-aryl-C₁₋₆-alkyle et un groupe protecteur ; et
- R₈ étant -(CH₂)₄-.

2. Revêtement selon la revendication 1 dans lequel b est au moins 0,25.

3. Revêtement selon l'une quelconque des revendications 1 et 2 dans lequel b est au moins 0,5.

4. Revêtement selon la revendication 1 dans lequel b est dans la plage de 0,25 à 0,75.

5. Revêtement selon la revendication 1, p = 0 et m + q = 1, m = 0,75, b étant 0,5 et a + b = 1, R₁ étant (CH₂)₈, R₃ étant (CH₃)-CH-CH₂-, R₅ étant hexyle, R₇ étant benzyle, R₈ étant -(CH₂)₄-.

6. Revêtement selon la revendication 1, m + p + q = 1, q = 0,25, p = 0,45 et m = 0,3, b étant 0,25 et a + b = 1, R₁ -(CH₂)₈ ; R₃ et R₄ respectivement, étant (CH₃)₂-CH-CH₂- ; R₅ étant (C₂-C₂₀)alkylène ; R₇ étant benzyle ; R₈ étant -(CH₂)₄- ; et R₆ étant choisi parmi des fragments bicycliques de 1,4:3,6-dianhydrohexitols de formule structurale (II).

7. Revêtement selon la revendication 1, m + p + q = 1, q = 0,25, p = 0,45 et m = 0,3, b étant 0,5, a + b = 1, R₁ étant- (CH₂) ₈- ; R₄ étant (CH₃)₂-CH-CH₂- ; R₇ étant benzyle ; R₈ étant -(CH₂)₄- ; et R₆ étant choisi parmi des fragments bicycliques de 1,4:3,6-dianhydrohexitols de formule structurale (II).

8. Revêtement selon la revendication 1, m + p + q = 1, q = 0,1, p = 0,30 et m = 0,6, b étant 0,5 et a + b = 1, R₁ -(CH₂)₄ -; R₃ et R₄ respectivement, étant (CH₃)₂-CH-CH₂- ; R₇ étant benzyle ; R₈ étant - (CH₂)₄- ; R₅ étant choisi dans le groupe constitué par (C₂-C₂₀)alkylène ; et R₆ étant choisi parmi des fragments bicycliques de 1,4:3,6-dianhydrohexitols de formule structurale (II).

9. Revêtement selon l'une quelconque des revendications 1 à 8 comprenant en outre un agent bioactif choisi dans le groupe des facteurs de croissance, des antibiotiques, des composés anti-inflammatoires, des composés anti-thrombogènes, des médicaments anti-claudication, des médicaments anti-arythmique, des médicaments anti-athérosclérotique, des antihistaminiques, des médicaments contre le cancer, des médicaments vasculaires, des médicaments ophtalmiques, des acides aminés, des vitamines, des hormones, des neurotransmetteurs, des neurohormones, des enzymes, des agents d'imagerie, des molécules de signalisation et des médicaments psychoactifs.

10. Dispositif implantable comprenant le revêtement selon l'une quelconque des revendications 1 à 9.

11. Dispositif implantable selon la revendication 10, le dispositif comprenant des pacemakers et des défibrillateurs cardiaques ; des connecteurs et des électrodes pour les précédents, des stimulateurs d'organes tels que des stimulateurs nerveux, de la vessie, du sphincter et du diaphragme, des prothèses, des tiges, des greffons vasculaires, des stents auto-expansibles, des stents expansibles par un ballon, des stents-greffons, des greffons, des cathéters, des valves de cœur artificiel et des dérivations de fluide cérébrospinal.

12. Revêtement selon la revendication 1, le rapport des groupes hydrophiles non protégés sur les groupes fonctionnels hydrophobes protégés variant de 0,3 à 1.

13. Revêtement selon la revendication 1, l'épaisseur étant de 2 à 15 µm.
